(19) Europäisches Patentamt
European Patent Office
Office européen des brevets

(11) Numéro de publication: **0 084 742**
**B1**

(12) **FASCICULE DE BREVET EUROPEEN**

(45) Date de publication du fascicule du brevet: **22.05.85**

(21) Numéro de dépôt: **82401215.7**

(22) Date de dépôt: **30.06.82**

(51) Int. Cl.[4]: **C 07 C 45/46**, C 07 C 49/807, C 07 C 49/83, C 07 C 49/84, C 07 C 49/813

(54) **Procédé d'acylation d'halogéno ou trihalogénométhylbenzènes.**

(30) Priorité: **21.01.82 FR 8200877**

(43) Date de publication de la demande: **03.08.83 Bulletin 83/31**

(45) Mention de la délivrance du brevet: **22.05.85 Bulletin 85/21**

(84) Etats contractants désignés: **AT BE CH DE FR GB IT LI LU NL SE**

(56) Documents cités:
**EP - A - 0 069 598**
**FR - A - 1 567 806**
**FR - A - 2 357 517**
**GB - A - 2 030 158**
**US - A - 3 387 035**
**US - A - 4 276 226**

(73) Titulaire: **RHONE-POULENC SPECIALITES CHIMIQUES, "Les Miroirs" 18, Avenue d'Alsace, F-92400 Courbevoie (FR)**

(72) Inventeur: **Desbois, Michel, 526, Chemin du Bois, F-69140-Rillieux (FR)**

(74) Mandataire: **Cazes, Jean-Marie et al, RHONE-POULENC RECHERCHES *Service Brevets Chimie et* Polymères 25, quai Paul Doumer, F-92408 Courbevoie Cedex (FR)**

**EP 0 084 742 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé d'acylation d'halogéno- ou de trihalogénométhylphénylbenzène.

On entend, au sens de la présente invention, par halogéno- ou trihalogénométhylbenzène un benzène comportant un substituant $CnX_1(X_2)_n(X_3)_n$ où n est égal à 0 ou 1, et où $X_1$, $X_2$, $X_3$ représentent un atome d'halogène.

L'invention concerne plus particulièrement la préparation de cétones par réaction d'acylation, c'est-à-dire par réaction de l'halogéno- ou du trihalogénométhylbenzène correspondant et d'un acide carboxylique, un précurseur ou un dérivé de cet acide.

On connaît dans l'art antérieur de tels procédés. C'est ainsi que G. Ollah décrit, dans «Friedel-Crafts and Related Reaction III», 1re partie, 1964, Interscience Publishers, cette réaction d'acylation en présence de catalyseurs comme $AlCl_3$, $AlBr_3$, $FeCl_3$, $SbCl_5$ en milieu solvant organique, le substrat pouvant être également le solvant.

Ces procédés présentent des inconvénients qui tiennent essentiellement à la nature du catalyseur. En effet, il est nécessaire d'utiliser une quantité importante de ce catalyseur: il faut utiliser plus de 1 mol de catalyseur, comme le chlorure d'aluminium, par mole de substrat, car le groupement carbonyle du réactif ou de la cétone formée complexe mole à mole le chlorure d'aluminium, d'où la nécessité de mettre en œuvre plus de 1 mol de ce dernier.

Cette quantité importante de $AlCl_3$ entraîne la mise en œuvre d'une grande quantité d'eau pour son élimination. De plus, sa récupération est impossible industriellement.

La réaction d'acylation est également connue dans l'art antérieur en présence de trifluorure de bore (voir G. Ollah cité ci-dessus).

Il est également connu, d'après les brevets US No 4276226 et GB No 2030158, de réaliser la réaction d'acylation en présence du couple catalytique acide chlorure/trifluorure de bore. Cette réaction n'a été mise en œuvre que sur des phénols, substrats benzéniques comportant un substituant activant, mais pas sur des substrats désactivés comme les halogénobenzènes ou trihalogénométhylbenzènes.

En effet, il était jusqu'à maintenant admis par l'homme de l'art que l'acylation de substrats désactivés comme les halogénobenzènes ne pouvait être obtenue que par la mise en œuvre d'un système catalytique puissant comme le chlorure d'aluminium, le trifluorure de bore et/ou l'acide fluorhydrique étant, dans les connaissances de l'homme de l'art, exclusivement réservés à l'acylation de dérivés activés comme les phénols.

La titulaire a découvert que le $BF_3$ en synergie avec l'acide fluorhydrique pouvait être mis en œuvre pour l'acylation de substrats désactivés.

Cela est d'une importance considérable sur le plan industriel car, on l'a vu plus haut, le chlorure d'aluminium présente des inconvénients techniques et économiques que ne présente pas le trifluorure de bore tel que mis en œuvre selon le procédé de l'invention.

Il est encore connu, d'après la demande de brevet européen No 69598 et le brevet anglais No 2102420, de préparer des phénylcétones par condensation de dérivés benzéniques monosubstitués par un halogène avec un dérivé acylé qui répond à la formule générale suivante:

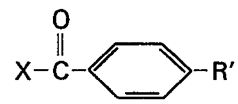

dans laquelle:

– R' représente Br, Cl, F, OH ou un groupe représenté par la formule générale suivante:

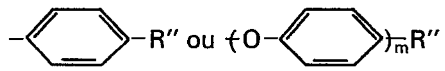

dans laquelle R'' représente Br, Cl, F ou OH, et m représente 1 ou 2,

– X représente Br, Cl, F, OH ou un groupe alcoxy en présence d'un catalyseur comprenant un acide de Lewis et un acide fort.

La présente invention concerne un procédé de préparation de phénylcétones par réaction d'un dérivé benzénique avec un acide carboxylique, aliphatique ou aromatique, un précurseur ou un dérivé de cet acide, en présence de trifluorure de bore en une quantité telle que la pression absolue de trifluorure de bore dans l'enceinte réactionnelle soit supérieure à 1 bar et en présence d'acide fluorhydrique comme solvant, caractérisé en ce que le dérivé benzénique est désactivé par au moins un groupe halogéné ou trihalogénométhylé; lorsque la réaction est réalisée avec le benzène monosubstitué par un halogène, le dérivé de l'acide carboxylique ne répond pas à la formule générale:

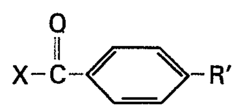

dans laquelle X représente un groupe choisi parmi F, Br, Cl, OH, alcoxy, R' représente un groupe choisi parmi F, Br, Cl, OH ou un groupe répondant à la formule générale:

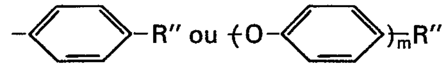

dans laquelle R'' représente F, Br, Cl ou OH et m représente 1 ou 2.

On connaît dans l'art antérieur une publication de MM. Buu-Hoi et Xuong dans «J. Org. Chem.», vol. 26, juillet 1961, pp. 2401 et 2402, qui décrit la synthèse de cétones phénoliques dérivées de di- et triphénols et d'α- et β-naphtol par condensation d'acides carboxyliques avec les phénols en présence du gaz produit par la réaction de l'oléum sur le fluoroborate de potassium. Ce gaz contient du trifluorure de bore et de l'acide fluorhydrique à côté d'autres composés comme l'acide fluorosulfonique.

Il est à noter que la réaction décrite dans cette publication a lieu en milieu solvant comme le xylène. Il est à noter de plus que le composé aromatique envisagé est un phénol, donc un benzène

comportant un substituant activant (OH). Un tel procédé n'est pas transposable à la préparation d'halogéno- ou de trihalogénométhylphénylcétones, dans la mesure où le solvant réagirait avec l'acide carboxylique, son précurseur ou son dérivé, l'halogéno- ou le trihalogénométhylbenzène étant moins activé que le xylène.

D'autre part, le gaz issu de la réaction de l'oléum sur le fluoroborate de potassium ne peut être assimilé au couple trifluorure de bore/acide fluorhydrique mis en œuvre dans le procédé selon l'invention. En effet, dans ce dernier, l'acide fluorhydrique à l'état liquide joue le rôle de solvant alors que, dans le document cité, il est sous forme gazeuse.

On entend, au sens de la présente invention, par halogénobenzène ou trihalogénométhylbenzène, d'une part, ces produits proprement dits et, d'autre part, leurs homologues correspondant à la présence d'un ou de plusieurs radicaux substituant le noyau benzénique.

Plus particulièrement encore, l'invention concerne la réaction des composés de formule:

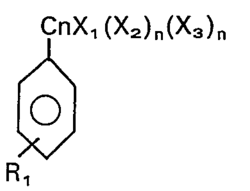

(I)

où:

– n est un nombre entier égal à 0 ou 1,

– $X_1$, $X_2$ et $X_3$, identiques ou différents, représentent le chlore, le brome, l'iode ou le fluor, et

– $R_1$ représente au moins un élément choisi parmi le groupe comprenant l'hydrogène, les radicaux alkyle et alcoxy ayant de 1 à 6 atomes de carbone, les radicaux phényle et phénoxy substitués par au moins un groupement plus désactivant que le groupement $CnX_1(X_2)_n(X_3)_n$, OH, Cl, Br, I et F.

Les radicaux $R_1$ phényle et phénoxy doivent être substitués par des groupements plus désactivants que le groupement $CnX_1(X_2)_n(X_3)_n$ pour que la réaction d'acylation intervienne sur le noyau benzénique porteur du groupement $CnX_1(X_2)_n(X_3)_n$. Dans le cas contraire, la réaction d'acylation se ferait sur le radical phényle ou phénoxy. On peut citer, comme exemples de groupements plus désactivants que le groupement $CnX_1(X_2)_n(X_3)_n$, les groupements $NO_2$, COOH, CN et cétones.

Quand n=1, on préfère mettre en œuvre des composés de formule I pour lesquels $X_1$, $X_2$ et $X_3$ sont identiques. Parmi ces derniers, on préfère ceux pour lesquels $X_1$, $X_2$ et $X_3$ représentent le fluor.

On peut citer, comme exemples de composés I: le chlorobenzène, le fluorobenzène, le bromobenzène, l'iodobenzène, le trifluorométhylbenzène, le difluorobromométhylbenzène, le trichlorométhylbenzène, le dichlorofluorométhylbenzène, le tribromométhylbenzène, le dibromofluorométhylbenzène, le triodométhylbenzène, l'o-, le m- et le p-fluorotoluène, l'o-, le m- et le p-dichlorobenzène, l'o-, le m- et le p-fluorophénol, l'o-, le m- et le p-fluorochlorobenzène, l'o-, le m- et le p-fluoroanisole, l'o-, le m- et le p-difluorobenzène, l'o-, le m- et le p-chlorotoluène, l'o-, le m- et le p-chloroanisole, le trifluorométhyl-4 chloro-4' biphényle, le trifluorométhyl-4 dichloro-2,4' diphényloxyde.

On entend, au sens de la présente invention, par acide carboxylique, le précurseur ou le dérivé de cet acide, tous les réactifs d'acylation classiques décrits dans l'art antérieur.

Selon un mode de réalisation particulier de la présente invention, l'acide carboxylique, le précurseur ou le dérivé de cet acide répond à la formule générale:

$$R_2-COX_4 \qquad (II)$$

dans laquelle:

– $R_2$ représente un radical aliphatique ou aromatique,

– $X_4$ représente un halogène, un groupe dérivant de l'anion d'un acide inorganique, OH, $OR_3$, $OCOR_4$, $NH_2$, $NHR_5$ ou $NR_6R_7$, où $R_3$, $R_4$, $R_5$, $R_6$ et $R_7$ représentent un radical aromatique ou aliphatique.

On peut citer comme exemples de groupes dérivant de l'anion d'un acide inorganique $ClO_4^-$ et $BF_4^-$.

L'invention est particulièrement bien adaptée à la mise en œuvre d'un composé de formule II dans laquelle $R_2$ représente un radical alkyle, phényle, alkylphényle, phénylalkyle ou phényle comportant au moins un substituant, par exemple un halogène, $NO_2$, CN, $NH_2$, COOH.

On peut citer, comme exemples de tels composés, le chlorure d'acétyle, l'acide acétique, l'anhydride acétique, le chlorure de benzoyle, l'acide benzoïque, l'anhydride benzoïque, le chlorure d'orthochlorobenzoyle, le chlorure de parachlorobenzoyle, le chlorure de parafluorobenzoyle, le fluorure de paratrifluorométhylbenzoyle, le fluorure d'orthotrifluorométhylbenzoyle, le chlorure de paranitrobenzoyle, l'acide paranitrobenzoïque, l'acide paraaminobenzoïque, le chlorure d'isobutyroyle, l'acide isobutyroïque, l'acide propanoïque, le chlorure de propanoyle, le chlorure de paratoluyle, le chlorure de parabenzylbenzoyle.

Le procédé selon l'invention est mis en œuvre de préférence en utilisant une quantité d'acide fluorhydrique telle que le rapport molaire de l'acide fluorhydrique à l'halogéno- ou au trihalogénométhylbenzène est compris entre 5 et 50. Encore plus préférentiellement, ce rapport est compris entre 10 et 30.

L'acide fluorhydrique mis en œuvre est de préférence anhydre. La mise en œuvre d'acide fluorhydrique aqueux entraînerait une consommation inutile de trifluorure de bore sous forme HF, $BF_3$, $H_2O$ ($H_3O^+BF_4^-$).

L'halogéno- ou le trihalogénométhylbenzène et l'acide carboxylique, son précurseur ou son dérivé sont utilisés en quantités sensiblement équimolaires. Un léger excès du premier peut cependant être souhaitable.

On préfère plus particulièrement utiliser une quantité de trifluorure de bore telle que la pression

absolue de $BF_3$ dans l'enceinte réactionnelle soit comprise entre 6 et 20 bar. Une pression supérieure à 20 bar n'est pas exclue de l'invention, mais n'apporte pas d'avantages particuliers. Plus la pression sera élevée, plus la vitesse de réaction augmentera. L'homme de l'art adaptera donc la pression à l'économie du procédé.

Le procédé de l'invention est de préférence mis en œuvre à une température comprise entre −20 et 150°C.

Les temps de réaction sont généralement compris entre quelques minutes et plusieurs heures.

Les phénylcétones obtenues selon le procédé de l'invention ont pour formule générale:

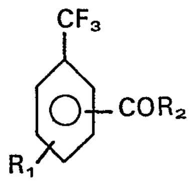

quand n=1, et

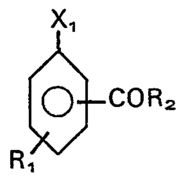

quand n=0, $X_1$, $R_1$ et $R_2$ ayant la signification précédente.

En effet, les groupements $CCl_3$, $CBr_3$, $CI_3$, $CF_2Br$, $CCl_2F$, $CBr_2F$, etc., se transforment en $CF_3$ lors de la réaction en milieu HF, les substituants Cl, Br et I pour leur part ne s'échangeant pas.

La position du groupement $COR_2$ par rapport aux groupements $CF_3$, $X_1$ et $R_1$ obéit aux règles de substitution bien connues du chimiste organicien.

Les phénylcétones obtenues selon le procédé de l'invention sont utiles comme intermédiaires de synthèse de composés ayant une activité pharmaceutique ou phytosanitaire.

On peut citer, comme exemples de composés pouvant être préparés par le procédé selon l'invention: fluoro-4 acétophénone, chloro-4 acétophénone, fluoro-2 méthyl-5 benzophénone, fluoro-3 méthyl-6 benzophénone, dichloro-2,4 benzophénone, dichloro-2,4' benzophénone, chloro-4 bromo-4 benzophénone, fluoro-4 bromo-4 benzophénone, difluoro-4,4' benzophénone, trifluorométhyl-4 fluoro-4' benzophénone, difluoro-4,4' méthyl-3 benzophénone, difluoro-4,4' méthoxy-3 benzophénone, fluoro-2 chloro-2' méthyl-5 benzophénone, fluoro-3 chloro-2' méthyl-6 benzophénone, fluoro-2 chloro-4' méthyl-5 benzophénone, fluoro-3 chloro-4' méthyl-6 benzophénone, fluoro-4 chloro-4' méthyl-3 benzophénone, trifluorométhyl-2, fluoro-2' méthyl-5 benzophénone, trifluorométhyl-2 fluoro-3' méthyl-6' benzophénone, fluoro-4 isobutyrophénone.

L'invention va être maintenant plus complètement décrite à l'aide des exemples qui vont suivre.

Ceux-ci ne sauraient être considérés comme limitant de façon quelconque l'invention.

*Exemple 1:*

Dans un réacteur inox de 250 ml muni d'un système d'agitation magnétique, on introduit, aux environs de 0°C, 100 ml de HF anhydre, 20,8 g (0,25 mol) de chlorure d'acétyle et 20 g (0,21 mol) de fluorobenzène. On ferme le réacteur, puis on introduit du trifluorure de bore ($BF_3$) gazeux jusqu'à la pression constante de 10 bar. On laisse alors réagir sous agitation pendant 23 h à la température ambiante. Après réaction, on décomprime le réacteur jusqu'à pression atmosphérique, puis on coule le mélange réactionnel sur 200 g de glace pilée. On extrait alors le mélange hétérogène ainsi obtenu par trois fois 200 $cm^3$ de chlorure de méthylène. Les phases organiques sont lavées par trois fois 200 $cm^3$ d'eau, 1 fois 200 $cm^3$ d'une solution aqueuse à 3% de potasse et 2 fois 200 $cm^3$ d'eau. La phase organique ainsi traitée est séchée sur sulfate de magnésium et le solvant est éliminé par distillation sous pression réduite. On recueille ainsi 26 g (rendement: 98%) de p-fluoroacétophénone à 90% de pureté.

*Exemple 2:*

On procède comme à l'exemple 1 avec les composés et conditions suivants:

- Acide fluorhydrique anhydre: 100 g
- Chlorobenzène: 22,5 g (0,2 mol)
- Chlorure d'acétyle: 23,6 g (0,3 mol)
- Trifluorure de bore: 6 bar à 20°C
- Température: 25°C
- Durée: 18 h

On recueille 29,8 g (rendement: 96,4%) de p-chloroacétophénone à 90% de pureté.

*Exemple 3:*

On procède comme à l'exemple 1 avec les composés et conditions suivants:

- Acide fluorhydrique anhydre: 100 g
- p-Fluorotoluène: 22 g (0,2 mol)
- Chlorure de benzoyle: 28,1 g (0,2 mol)
- Trifluorure de bore: 6 bar à 20°C
- Température: 20°C
- Durée: 3 h

On recueille 37,1 g (rendement: 86,7%) d'un mélange de fluoro-2, méthyl-5 benzophénone et de fluoro-3, méthyl-6 benzophénone.

*Exemple 4:*

On procède comme à l'exemple 1 avec les composés et conditions suivants:

- Acide fluorhydrique anhydre: 100 g
- m-Dichlorobenzène: 29,4 g (0,2 mol)
- Chlorure de benzoyle: 28,1 g (0,2 mol)
- Trifluorure de bore: 10 bar à 20°C
- Température: 100°C
- Durée: 18 h

On recueille 44,7 g (rendement: 89%) de dichloro-2,4 benzophénone à 95% de pureté.

*Exemple 5:*

On procède comme à l'exemple 1 avec les composés et conditions suivants:

- Acide fluorhydrique anhydre: 100 g

– Chlorobenzène: 22,5 g (0,2 mol)
– Chlorure d'o-chlorobenzoyle: 35 g (0,2 mol)
– Trifluorure de bore: 10 bar à 20°C
– Température: 20°C
– Durée: 23 h

On recueille 49,1 g (rendement: 97,8%) de dichloro-2,4' benzophénone à 97% de pureté.

*Exemple 6:*

On procède comme à l'exemple 1 avec les composés et conditions suivants:
– Acide fluorhydrique anhydre: 100 g
– Trifluorométhylbenzène: 29,2 g (0,2 mol)
– Chlorure de p-fluorobenzoyle: 31,7 g (0,2 mol)
– Trifluorure de bore: 10 bar à 20°C
– Température: 80°C
– Durée: 19 h

On recueille 49,4 g (rendement: 92,1%) de trifluorométhyl-4, fluoro-4' benzophénone à 70% de pureté.

*Exemple 7:*

On procède comme à l'exemple 1 avec les composés et conditions suivants:
– Acide fluorhydrique anhydre: 100 g
– o-Fluorotoluène: 22 g (0,2 mol)
– Chlorure de p-fluorobenzoyle: 31,7 g (0,2 mol)
– Trifluorure de bore: 10 bar à 20°C
– Température: 20°C
– Durée: 19 h

On recueille 40,6 g (rendement: 87,5%) de difluoro-4,4' méthyl-3 benzophénone à 97% de pureté.

*Exemple 8:*

On procède comme à l'exemple 1 avec les composés et conditions suivants:
– Acide fluorhydrique anhydre: 100 g
– o-Fluoroanisole: 25,8 g (0,2 mol)
– Chlorure de p-fluorobenzoyle: 31,7 g (0,2 mol)
– Trifluorure de bore: 6 bar à 20°C
– Température: 10°C
– Durée: 1 h

On recueille 47,8 g (rendement: 96,4%) de difluoro-3,4' méthoxy-4 benzophénone à 97% de pureté.

*Exemple 9:*

On procède comme à l'exemple 1 avec les composés et conditions suivants:
– Acide fluorhydrique anhydre: 100 g
– Fluorobenzène: 19,2 g (0,2 mol)
– Anhydride acétique: 10,2 g (0,1 mol)
– Trifluorure de bore: 10 bar à 20°C
– Température: 25°C
– Durée: 4 h

On recueille 24 g (rendement: 87%) de p-fluoroacétophénone à 98% de pureté.

*Exemple 10:*

On procède comme à l'exemple 1 avec les composés et conditions suivants:
– Acide fluorhydrique anhydre: 100 g
– p-Fluorotoluène: 22 g (0,2 mol)
– Anhydride benzoïque: 22,6 g (0,1 mol)
– Trifluorure de bore: 10 bar à 20°C
– Température: 30°C
– Durée: 3 h

On recueille 36,7 g (rendement: 85,7%) d'un mélange de fluoro-2 méthyl-5 benzophénone et de fluoro-3 méthyl-6 benzophénone.

*Exemple 11:*

On procède comme à l'exemple 1 avec les composés et conditions suivants:
– Acide fluorhydrique anhydre: 100 g
– Fluorobenzène: 19,2 g (0,2 mol)
– Acide acétique: 12 g (0,2 mol)
– Trifluorure de bore: 10 bar à 20°C
– Température: 50°C
– Durée: 5 h

On recueille 17 g (rendement: 61,5%) de p-fluoroacétophénone à 99,5% de pureté.

*Exemple 12:*

On procède comme à l'exemple 1 avec les composés et conditions suivants:
– Acide fluorhydrique anhydre: 100 g
– Fluorobenzène: 19,2 g (0,2 mol)
– Acide benzoïque: 24,4 g (0,2 mol)
– Trifluorure de bore: 15 bar à 20°C
– Température: 50°C
– Durée: 4½ h

On recueille 36,3 g (rendement: 90,7%) de fluoro-4 benzophénone à 99% de pureté.

*Exemple 13:*

On procède comme à l'exemple 1 avec les composés et conditions suivants:
– Acide fluorhydrique anhydre: 100 g
– p-Fluorotoluène: 22 g (0,2 mol)
– Acide benzoïque: 24,4 g (0,2 mol)
– Trifluorure de bore: 10 bar à 20°C
– Température: 50°C
– Durée: 4 h

On recueille 34,4 g (rendement: 80,4%) d'un mélange de fluoro-2, méthyl-5 benzophénone et de fluoro-3, méthyl-6 benzophénone.

*Exemple 14:*

On procède comme à l'exemple 1 avec les composés et conditions suivants:
– Acide fluorhydrique anhydre: 100 g
– Fluorobenzène: 19,2 g (0,2 mol)
– Fluorure de p-trifluorométhylbenzoyle: 38,4 g (0,2 mol)
– Trifluorure de bore: 6 bar à 20°C
– Température: 20°C
– Durée: 4 h

On recueille 48 g (rendement: 89,5%) de trifluorométhyl-4, fluoro-4' benzophénone à 99% de pureté.

*Exemple 15:*

On procède comme à l'exemple 1 avec les composés et conditions suivants:
– Acide fluorhydrique anhydre: 100 g

– p-Fluorotoluène: 22 g (0,2 mol)

– Fluorure d'o-trifluorométhylbenzoyle: 38,4 g (0,2 mol)

– Trifluorure de bore: 6 bar à 20°C

– Température: 23°C

– Durée: 1 h

On recueille 42,6 g (rendement: 75%) d'un mélange de trifluorométhyl-2, fluoro-2', méthyl-5' benzophénone et de trifluorométhyl-2, fluoro-3', méthyl-6' benzophénone.

*Exemple 16:*

On procède comme à l'exemple 1 avec les composés et conditions suivants:

– Acide fluorhydrique anhydre: 100 g

– Chloro-3 fluorobenzène: 13 g (0,1 mol)

– Chlorure de p-fluorobenzoyle: 15,9 g (0,1 mol)

– Trifluorure de bore: 6 bar à 20°C

– Température: 100°C

– Durée: 13 h

On recueille 23,4 g (rendement: 81%) d'un mélange de difluoro-4,4', chloro-2 benzophénone et de difluoro-2,4' chloro-4 benzophénone dans la proportion de 55/45.

*Exemple 17:*

On procède comme à l'exemple 1 avec les composés et conditions suivants:

– Acide fluorhydrique anhydre: 100 g

– Fluoro-3 phénol: 23,4 g (0,2 mol)

– Chlorure d'o-chlorobenzoyle: 35 g (0,2 mol)

– Trifluorure de bore: 6 bar à 20°C

– Température: 0°C

– Durée: 1 h

On recueille 48,2 g (rendement: 96,2%) d'un mélange de fluoro-4 chloro-2' hydroxy-2 benzophénone et de fluoro-2 chloro-2' hydroxy-4 benzophénone.

Dans cet exemple, le lavage par la solution aqueuse de potasse à 3% a été supprimé afin de ne pas extraire le phénol formé.

*Exemple 18:*

On procède comme à l'exemple 1 avec les composés et conditions suivants:

– Acide fluorhydrique anhydre: 100 g

– Trichlorométhylbenzène: 39,1 g (0,2 mol)

– Chlorure de p-fluorobenzoyle: 31,7 g (0,2 mol)

– Trifluorure de bore: 10 bar à 20°C

– Température: 80°C

– Durée: 20 h

On recueille 42 g (rendement: 78%) de trifluorométhyl-4 fluoro-4' benzophénone à 65% de pureté.

On note, pendant la réaction, une augmentation de pression due au départ d'acide chlorhydrique provenant de l'échange Cl-F.

*Exemple 19:*

On procède comme à l'exemple 1 avec les composés et conditions suivants:

– Acide fluorhydrique anhydre: 100 g

– Fluoro-4 phénol: 23,4 g (0,2 mol)

– Chlorure de parachlorobenzoyle: 35 g (0,2 mol)

– Trifluorure de bore: 6 bar à 20°C

– Température: 80°C

– Durée: 20 h

On recueille 45 g (rendement: 80%) de fluoro-3 chloro-4' hydroxy-2 benzophénone à 92% de pureté.

Dans cet exemple, le lavage par la solution aqueuse de potasse à 3% a été supprimé afin de ne pas extraire le phénol formé.

*Exemple 20:*

On procède comme à l'exemple 1 avec les composés et conditions suivants:

– Acide fluorhydrique anhydre: 100 g

– Difluorobromométhylbenzène: 41,4 g (0,2 mol)

– Chlorure de p-fluorobenzoyle: 31,7 g (0,2 mol)

– Trifluorure de bore: 10 bar à 20°C

– Température: 100°C

– Durée: 15 h

On recueille 48 g (rendement: 89%) de trifluorométhyl-4 fluoro-4' benzophénone à 72% de pureté.

On note, pendant la réaction, une augmentation de pression due au départ d'acide bromhydrique provenant de l'échange Br-F.

**Revendications**

1. Procédé de préparation de phénylcétones par réaction d'un dérivé benzénique avec un acide carboxylique, aliphatique ou aromatique, un précurseur ou un dérivé de cet acide, en présence de trifluorure de bore en une quantité telle que la pression absolue de trifluorure de bore dans l'enceinte réactionnelle soit supérieure à 1 bar et en présence d'acide fluorhydrique comme solvant, caractérisé en ce que le dérivé benzénique est désactivé par au moins un groupe halogéné ou trihalogénométhylé; lorsque la réaction est réalisée avec le benzène monosubstitué par un halogène, le dérivé de l'acide carboxylique ne répond pas à la formule générale:

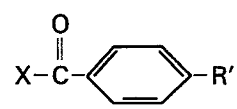

dans laquelle X représente un groupe choisi parmi F, Br, Cl, OH, alcoxy; R' représente un groupe choisi parmi F, Br, Cl, OH ou un groupe répondant à la formule générale

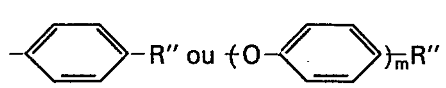

dans laquelle R" représente F, Br, Cl ou OH et m représente 1 ou 2.

2. Procédé selon la revendication 1, caractérisé en ce que le dérivé benzénique répond à la formule générale:

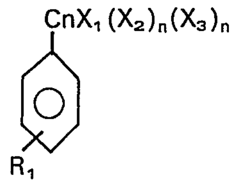

(I)

où:

– n est un nombre entier égal à 0 ou 1,

– $X_1$, $X_2$ et $X_3$, identiques ou différents, représentent le chlore, le brome, l'iode ou le fluor, et

– $R_1$ représente au moins un élément choisi parmi le groupe comprenant l'hydrogène, les radicaux alkyle et alcoxy ayant de 1 à 6 atomes de carbone, les radicaux phényle et phénoxy substitués par au moins un groupement plus désactivant que le groupement $CnX_1(X_2)_n(X_3)_n$, OH, Cl, Br, I et F.

3. Procédé selon l'une des revendications précédentes, caractérisé en ce que l'acide carboxylique, le précurseur ou le dérivé de cet acide répond à la formule générale:

$$R_2-COX_4 \qquad (II)$$

dans laquelle:

– $R_2$ représente un radical aliphatique ou aromatique; lorsque la réaction est réalisée avec le benzène monosubstitué par un halogène, $R_2$ ne répond pas à la formule générale:

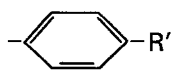

dans laquelle R' représente un groupe choisi parmi F, Br, Cl, OH, ou un groupe répondant à la formule générale

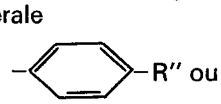

ou

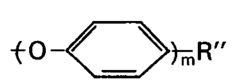

dans laquelle R'' représente F, Cl, Br ou OH et m représente 1 ou 2,

– $X_4$ représente un halogène, un groupe dérivant de l'anion d'un acide inorganique, OH, $OR_3$, $OCOR_4$, $NH_2$, $NHR_5$ ou $NR_6R_7$, où $R_3$, $R_4$, $R_5$, $R_6$ et $R_7$ représentent un radical aromatique ou aliphatique.

4. Procédé selon la revendication 3, caractérisé en ce que $R_2$ représente un radical alkyle, phényle, alkylphényle, phénylalkyle ou phényle comportant au moins un substituant halogène, $NO_2$, CN, $NH_2$, COOH.

5. Procédé selon l'une des revendications précédentes, caractérisé en ce qu'on utilise une quantité d'acide fluorhydrique telle que le rapport molaire de l'acide fluorhydrique à l'halogéno- ou au trihalogénométhylbenzène est compris entre 5 et 50.

6. Procédé selon l'une des revendications précédentes, caractérisé en ce que l'acide fluorhydrique mis en œuvre est de l'acide fluorhydrique anhydre.

7. Procédé selon l'une des revendications précédentes, caractérisé en ce que l'halogéno- ou le trihalogénométhylbenzène et l'acide carboxylique, son précurseur ou son dérivé sont utilisés en quantités sensiblement équimolaires.

8. Procédé selon l'une des revendications précédentes, caractérisé en ce qu'on utilise une quantité de trifluorure de bore telle que la pression absolue de $BF_3$ dans l'enceinte réactionnelle soit comprise entre 6 et 20 bar.

9. Procédé selon l'une des revendications précédentes, caractérisé en ce qu'on opère à une température comprise entre −20 et 150°C.

## Patentansprüche

1. Verfahren zur Herstellung von Phenylketonen durch Reaktion eines Benzolderivats mit einer aliphatischen oder aromatischen Carbonsäure, einem Vorläufer oder einem Derivat dieser Säure in Gegenwart von Bortrifluorid in einer solchen Menge, dass der absolute Druck des Bortrifluorids in dem Reaktionsgefäss über 1 bar liegt, und in Gegenwart von Fluorwasserstoffsäure als Lösungsmittel, dadurch gekennzeichnet, dass das Benzolderivat durch wenigstens eine Halogen- oder Trihalogenmethylgruppe desaktiviert wird; wenn die Reaktion mit einem einfach halogensubstituierten Benzol durchgeführt wird, entspricht das Carbonsäurederivat nicht der allgemeinen Formel:

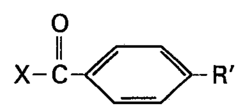

in der X eine aus F, Br, Cl, OH oder der Alkoxygruppe ausgewählte Gruppe ist; R' bedeutet eine Gruppe aus F, Br, Cl, OH oder eine Gruppe der allgemeinen Formel

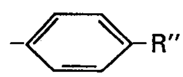

oder

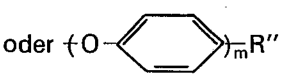

in der R'' F, Br, Cl oder OH bedeutet und m 1 oder 2 ist.

2. Verfahren nach Anspruch 1, dadurch gekennzeichnet, dass das Benzolderivat die allgemeine Formel besitzt:

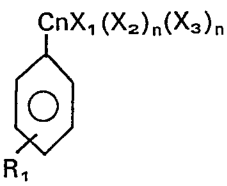

(I)

in der

– n=0 oder 1 ist,

– $X_1$, $X_2$ und $X_3$, gleich oder verschieden, Chlor, Brom, Jod oder Fluor bedeuten, und

– $R_1$ wenigstens ein Element aus der Gruppe ist, die den Wasserstoff, die Alkyl- und Alkoxyradikale mit 1 bis 6 Kohlenstoffatomen und die mit wenigstens einer stärker desaktivierenden Gruppe als die Gruppe $CnX_1(X_2)_n(X_3)_n$, OH, Cl, Br, J und Fluor substituierten Phenyl- und Phenoxyradikale umfasst.

3. Verfahren nach einem der vorangehenden Ansprüche, dadurch gekennzeichnet, dass die Carbonsäure, der Vorläufer oder das Derivat dieser Säure die allgemeine Formel besitzt:

$$R_2-COX_4 \qquad (II)$$

in der

– $R_2$ ein aliphatisches oder aromatisches Radikal bedeutet; wenn die Reaktion mit dem durch ein Halogen einfach substituierten Benzol durchgeführt wird, entspricht $R_2$ nicht der allgemeinen Formel:

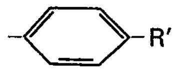

in der R' einen Rest, ausgewählt aus der Gruppe F, Br, Cl, OH, oder eine Gruppe, die die allgemeine Formel besitzt

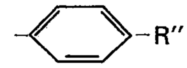 oder 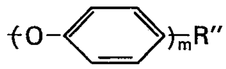

in der R'' F, Cl, Br oder OH und m 1 oder 2 ist, bedeutet,

– $X_4$ ein Halogen, eine von dem Anion einer anorganischen Säure abgeleitete Gruppe, OH, $OR_3$, $OCOR_4$, $NH_2$, $NHR_5$ oder $NR_6R_7$ ist, wobei $R_3$, $R_4$, $R_5$, $R_6$ und $R_7$ ein aromatisches oder aliphatisches Radikal bedeuten.

4. Verfahren nach Anspruch 3, dadurch gekennzeichnet, dass $R_2$ ein Alkyl-, Phenyl-, Alkylphenyl-, Phenylalkyl- oder ein Phenylradikal ist, das wenigstens einen Halogen-, $NO_2$-, CN-, $NH_2$-, COOH-Substituenten trägt.

5. Verfahren nach einem der vorangehenden Ansprüche, dadurch gekennzeichnet, dass man eine derartige Menge an Fluorwasserstoffsäure einsetzt, dass das Molverhältnis von Fluorwasserstoffsäure zu dem Halogen- oder Trihalogenmethylbenzol zwischen 5 und 50 liegt.

6. Verfahren nach einem der vorangehenden Ansprüche, dadurch gekennzeichnet, dass die eingesetzte Fluorwasserstoffsäure wasserfreie Fluorwasserstoffsäure ist.

7. Verfahren nach einem der vorangehenden Ansprüche, dadurch gekennzeichnet, dass das Halogen- oder Trihalogenmethylbenzol und die Carbonsäure, ihr Vorläufer oder ihr Derivat in ungefähr äquimolaren Mengen eingesetzt werden.

8. Verfahren nach einem der vorangehenden Ansprüche, dadurch gekennzeichnet, dass man eine derartige Menge Bortrifluorid einsetzt, dass der absolute Druck des Bortrifluorids in dem Reaktionsgefäss zwischen 6 und 20 bar liegt.

9. Verfahren nach einem der vorangehenden Ansprüche, dadurch gekennzeichnet, dass man bei einer Temperatur zwischen −20 und 150°C arbeitet.

## Claims

1. Process for preparing phenyl ketones by reaction of a benzene derivative with an aliphatic or aromatic carboxylic acid, a precursor or a derivative of this acid, in the presence of boron trifluoride in such quantity that the absolute pressure of boron trifluoride in the reaction enclosure is greater than 1 bar and in the presence of hydrofluoric acid as a solvent, characterised in that the benzene derivative is deactivated by means of at least one halo or trihalomethyl group; when the reaction is carried out with benzene monosubstituted by a halogen, the derivative of the carboxylic acid does not correspond to the general formula:

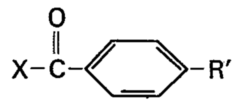

in which X denotes a group chosen from F, Br, Cl, OH, or alkoxy; R' denotes a group chosen from F, Br, Cl, OH or a group corresponding to the general formula:

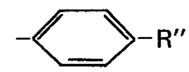 or 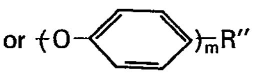

in which R'' denotes F, Br, Cl or OH and m denotes 1 or 2.

2. Process according to Claim 1, characterised in that the benzene derivative corresponds to the general formula:

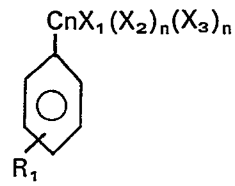

(I)

where:

– n is an integer equal to 0 or 1,

– $X_1$, $X_2$ and $X_3$, which are identical or different, denote chlorine, bromine, iodine or fluorine, and

– $R_1$ denotes at least one member chosen from the group comprising hydrogen, alkyl and alkoxy radicals containing from 1 to 6 carbon atoms, the phenyl and phenoxy radicals substituted by at least one group which is more deactivating than the group $CnX_1(X_2)_n(X_3)_n$, OH, Cl, Br, I and F.

3. Process according to either of the preceding claims, characterised in that the carboxylic acid, the precursor or the derivative of this acid corresponds to the general formula:

$$R_2-COX_4 \qquad (II)$$

in which:

– $R_2$ denotes an aliphatic or aromatic radical; when the reaction is carried out with benzene monosubstituted by a halogen, $R_2$ does not correspond to the general formula:

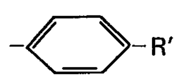

in which R' denotes a group chosen from F, Br, Cl, OH or a group corresponding to the general formula

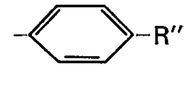 or 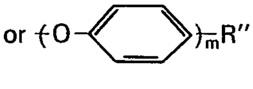

in which R'' denotes F, Cl, Br or OH and m denotes 1 or 2,

– $X_4$ denotes a halogen, a group derived from the anion of an inorganic acid, OH, $OR_3$, $OCOR_4$, $NH_2$, $NHR_5$ or $NR_6R_7$, where $R_3$, $R_4$, $R_5$, $R_6$ and $R_7$ denote an aromatic or aliphatic radical.

4. Process according to Claim 3, characterised in that $R_2$ denotes an alkyl, phenyl, alkylphenyl, or

phenylalkyl radical, or a phenyl radical containing at least one substituent halogen, $NO_2$, CN, $NH_2$, or COOH.

5. Process according to any one of the preceding claims, characterised in that a quantity of hydrofluoric acid is employed such that the molar ratio of hydrofluoric acid to the halo or trihalomethylbenzene is between 5 and 50.

6. Process according to one of the preceding claims, characterised in that the hydrofluoric acid employed is anhydrous hydrofluoric acid.

7. Process according to one of the preceding claims, characterised in that the halo or trihalomethylbenzene and the carboxylic acid, its precursor or its derivative are employed in substantially equimolar quantities.

8. Process according to one of the preceding claims, characterised in that a quantity of boron trifluoride is employed such that the absolute pressure of $BF_3$ in the reaction enclosure is between 6 and 20 bar.

9. Process according to one of the preceding claims, characterised in that it is operated at a temperature of between −20 and 150°C.